# EUROPEAN PATENT SPECIFICATION

(11) **EP 1 689 410 B1**
(45) Date of publication and mention of the grant of the patent: **21.05.2008**
(21) Application number: 04803177.7
(22) Date of filing: 16.11.2004
(51) Int. Cl.: A61K 31/565, A61P 9/00, A61P 9/02, A61P 9/04, A61P 9/08, A61P 9/10, A61P 9/12

(54) **PREVENTION AND TREATMENT OF HYPERTENSIVE HEART DISEASES BY THE SELECTIVE ESTROGENS 8BETA-VINYL-ESTRA-1,3,5(10)-TRIEN-3,17BETA-DIOL AND 17BETA-FLUOR-9ALPHA-VINYL-ESTRA-1,3,5(10)-TRIEN-3,16ALPHA-DIOL**
PRÄVENTION UND BEHANDLUNG VON HYPERTONEN HERZERKRANKUNGEN MIT DEN SELEKTIVEN ÖSTROGENEN 8BETA-VINYL-ESTRA-1,3,5(10)-TRIEN-3,17BETA-DIOL UND 17BETA-FLUOR-9ALPHA-VINYL-ESTRA-1,3,5(10)-TRIEN-3,16ALPHA-DIOL
PREVENTION ET TRAITEMENT DE MALADIES CARDIAQUES HYPERTENSIVES A L'AIDE DES OESTROGENES SELECTIFS 8BETA-VINYL-ESTRA-1,3,5(10)-TRIEN-3,17BETA-DIOL ET 17BETA-FLUOR-9ALPHA-VINYL-ESTRA-1,3,5(10)-TRIEN-3,16ALPHA-DIOL

(30) Priority: 26.11.2003 EP 03090406
(43) Date of publication of application: 16.08.2006
(73) Proprietor: Bayer Schering Pharma Aktiengesellschaft, 13353 Berlin (DE)
(72) Inventor: HEGELE-HARTUNG, Christa, 45470 Mülheim a.d. Ruhr (DE); FRITZEMEIER, Karl-Heinrich, 13505 Berlin (DE); PETERS, Olaf, 99891 Tabarz (DE); PELZER, Theo, 97078 Würzburg (DE); NEYSES, Ludwig, Didsbury Greater Manchester M20 5HJ (GB)
(86) International application number: PCT/EP2004/013121
(87) International publication number: WO 2005/051401

(56) References cited:
- WO-A-03/104253
- DE-A1- 10 019 167
- GB-A- 2 374 412
- MOERS A; SCHREZENMEIR J: "Palmitic acid but not stearic acid inhibits NO-production in endothelial cells" EXPERIMENTAL AND CLINICAL ENDOCRINOLOGY AND DIABETES, vol. 105, no. 2, 1997, pages 78-80, XP009049890
- CATALANO M; LIBRETTI A: "Captopril for the treatment of patients with hypertension and peripheral vascular disease" ANGIOLOGY, vol. 36, no. 5, 1985, pages 293-296, XP009049918
- [Online] Retrieved from the Internet: URL:http://www.who.int/classifications/app s/icd/icd10online/> [retrieved on 2007-07-11]

## Description

The invention relates to the prevention and treatment of hypertensive heart diseases.

### Summary of Invention

The invention relates to the prevention and treatment of one or more of the conditions selected from the group of (1) hypertension, (2) cardiac hypertrophy and (3) heart failure using 8β-Vinyl-estra-1,3,5(10)-trien-3,17p-diol and 17β-Fluor-9α-vinyl-estra-1,3,5(10)-trien-3,16α-diol, two steroidal estrogen receptor ligands, exhibiting selectivity for estrogen receptor beta (ERβ), and prodrugs of 8β-Vinyl-estra-1,3,5(10)-trien-3,17β-diol and 17β-Fluor-9α-vinyl-estra- 1,3,5(10)-trien-3,16α-diol.

The invention is based on the finding that the ER-β selective agonists 8β-Vinyl-estra-1,3,5(10)-trien-3, 17β-diol and 17β-Fluor-9α-vinyl-estra-1 , 3, 5(10)-trien-3, 16α-diol
(i) lower systolic, mean and diastolic blood pressure,
(ii) prevent the development of cardiac hypertrophy and
(iii) increase cardiac output
in a preclinical model of hypertension, namely spontaneously hypertensive rats (SHR).

Whereas abnormal vascular function and hypertension has been observed in ERβ-deficient mice (Zhu Y et al. 2002, Science 295: 505-508), these observations indicating an essential role for ERβ in the regulation of vascular function and blood pressure, the demonstration for the two ERβ agonists 8β-Vinyl-estra-1 ,3,5(10)-trien-3,1 7β-diol and 1 7β-Fluor-9α-vinyl-estra-1 ,3,5(1 0)-trien-3,1 6α-diol
(i) to lower systolic, mean and diastolic blood pressure,
(ii) to prevent the development of cardiac hypertrophy and
(iii) to increase cardiac output
in a preclinical model of hypertension, spontaneously hypertensive rats (SHR), is new.

The phenotype of disturbed vascular function in ERβ knockout mice was the basis for the patent application GB 2374412 A by Gustafsson JA and Bian Z (Karo Bio AB, Sweden) filed by April 4 2001. The inventors observed an elevation of mean arterial pressure (MAP) in both male and female ERβ-knockout mice with an age of more than 5 months compared to wild type (wt) littermates. Cardiac mass in the respective ERβ deficient mice was not different from wt littermates and pathological alterations such as hypertrophy or fibrosis were not detected in neither heart, lung nor kidney. Whereas the inventors claim the use of ERβ modulating compounds for modulating blood-pressure and in particular for treating hypertension, a study with ERβ selective agonists documenting these effects has not been published, so far.

In view of the phenotype of the ERβ deficient mice described in GB 2374412 providing no evidence for pathological alterations like cardiac hypertrophy or fibrosis, the present inventors' finding that the ERβ agonists 8β-Vinyl-estra-1,3,5(10)-trien-3,17β-diol (WO0177139) and 17β-Fluor-9α-vinyl-estra-1,3,5(10)-trien-3,16α-diol (PCT/EP03/06172) used in the present study inhibit cardiac hypertrophy in SHR is unexpected and new.
Furthermore, it has been found that the two ERβ agonists 8β-Vinyl-estra-1,3,5(10)-trien-3,17β-diol and 17β-Fluor-9α-vinyl-estra-1,3,5(10)-trien-3, 16α-diol investigated are more effective than 17β-estradiol and the selective ERα agonist 3,17β-Dihydroxy-19-nor-17α-pregna-1,3,5(10)-triene-21,16α-lactone (WO 02/26763) in lowering blood pressure in SHR.

These observations provide the basis to propose the use of the two ERβ agonists 8β-Vinyl-estra-1,3,5(10)-trien-3,17β-diol and 17β-Fluor-9α-vinyl-estra-1,3,5(10)-trien-3,16α-diol for the prevention and/or treatment of
(1) hypertension,
(2) cardiac hypertrophy and
(3) heart failure.

### Background

Hypertension is one of the key factors determining cardiovascular morbidity and mortality in humans (Vasan RS, Larson MG, Leip EP, Evans JC, O'Donnell CJ, Kannel WB, Levy D. Impact of high-normal blood pressure on the risk of cardiovascular disease. N Engl J Med 2001; 345:1291-1297). The prevalence of hypertension is significantly lower in pre-menopausal women compared to age matched men (Corrao JM, Becker RC, Ockene IS, Hamilton GA. CHD risk factors in women. Cardiology 1990; 77:8 - 24). These sex differences are no longer observed after menopause (August P, Oparil S. Hypertension in women. Jclin Endocrinol Metabol 1999; 84:1826 - 1866) as a consequence of the estrogen deficiency caused by the decline of ovarian function. Estrogen deficiency may also be caused by pharmacological intervention e.g. by GnRH agonists or antagonists, aromatase inhibitors or antiestrogens used for the therapy of estrogen dependent cancers (e.g. breast and prostate cancer), endometriosis and other diseases. The association of clinical symptoms of estrogen deficiency with this type of therapies is well documented. Thus, the therapy with GnRH analogues is associated with the induction of hot flashes and a reduction in bone mineral density (Sagsveen M. et al. 2003, Cochrane Database Syst Rev 4: CD001297). Also for aromatase inhibitors negative effects of the suppression of estrogen action as the induction of vasomotor symptoms and a decrease in bone mineral density have been described (Wickman S. et al. 2003, J. Clin. Endocrinol Metab. 88: 3785-93). Estrogens are known to exert beneficial effects on the vascular wall including the up-regulation of endothelial nitric-oxide synthase (Hayashi T, Yamada K, Esaki T, Kuzuya M, Satake S, Ishikawa T, Hidaka H, Iguchi A. Estrogen increases endothelial nitric oxide by a receptor mediated system. Biochem Biophys Res Comm 1995; 214:847-855; MacRitchie AN, Jun SS, Chen Z, German Z, Yuhanna IS, Sherman TS, Shaul PW. Estrogen upregulates endothelial nitric oxide synthase gene expression in fetal pulmonary artery endothelium. Circ Res. 1997;81:355-62) and angiotensin-1 receptor expression (Nickenig G, Baumer AT, Grohe C, Kahlert S, Strehlow K, Rosenkranz S, Stablein A, Beckers F, Smits JF, Daemen MJ, Vetter H, Boehm M. Estrogen modulates AT1 receptor gene expression in vitro and in vivo. Circulation 1998; 97:2197-2201). Estradiol as a natural, non-selective ERα and ERβ agonist, lowers blood pressure in post-menopausal women (Modena MG, Molinari R, Muia N, Castelli A, Pala F, Rossi R. Double blind randomized placebo controlled study of transdermal estrogen replacement therapy on hypertensive postmenopausal women. Am J Hypertens 1999; 12:1000-1008; Szekas B, Vajo Z, Acs N, Hada P, Csuzi L, Bezeredi J, Magyar Z, Brinton EA. HRT reduces mean 24 hr blood pressure and its variability in postmenopausal women with treated hypertension. Menopause 2000; 7:31-35). Hypertension increases cardiac workload which leads to an adaptive increase of myocardial mass (myocardial hypertrophy). Although cardiac hypertrophy per se represents a compensatory mechanism, it is nevertheless an established and independent risk factor for the development of heart failure and sudden cardiac death (Levy D, Garrison RJ, Savage DD, Kannel WB, Castelli WP. Prognostic implications of echocardiographically determined left ventricular mass in the Framingham Heart Study. N Engl J Med 1990; 322:1561-1566). The principle finding, that gender itself acts as a key component in determining the extent of left ventricular hypertrophy has been reported in several population based studies including the Framingham. population and patients with aortic stenosis (Carroll JD, Carroll EP, Feldman T, Ward DM , Lang RM, McGaughey D and Karp RB. Sex-associated differences in left ventricular function in aortic stenosis of the elderly. Circulation 1992; 86:1099-1107; Krumholz HM, Larson M, Levy D. Sex differences in cardiac adaptation to isolated systolic hypertension. Am J Cardiol 1993; 72:310-313; Dannenberg AL, Levy D, Garrison RJ. Impact of age on echocardiographic left ventricular mass in a healthy population (the Framingham study). Am J Cardiol 1989; 64:1066-1068). The direct link between cardiac hypertrophy and sex hormone levels has recently been established in animal models following aortic constriction or the development of hypertension in female SHR rats (Wallen WJ, Cserti Ch, Belanger MP, Wittnich C. Gender-differences in myocardial adaptation to afterload in normotensive and hypertensive rats. Hypertension 2000; 36:774-779; van Eickels M, Grohe C, Cleutjens JP, Janssen BJ, Wellens HJ, Doevendans PA. 17beta-estradiol attenuates the development of pressure-overload hypertrophy. Circulation. 2001; 18;104:1419-1423; Pelzer T, de Jager T, Muck J, Stimpel M, Neyses L. Oestrogen action on the myocardium in vivo: specific and permissive for angiotensin-converting enzyme inhibition. J Hypertens 2002; 20:1001-1006). Myocardial hypertrophy, coronary artery disease as well as valvular heart disease, finally lead to heart failure, which is defined as the inability of the heart to maintain cardiac output at a sufficient level to provide peripheral tissues with the required amount of oxygenated blood. Advanced stages of heart failure carry a poor prognosis that is comparable to advanced stages of malignant disease and represents the single most important cause of cardiovascular mortality in industrial countries (Haddy FJ. Heart failure - incidence and survival. N Engl J Med. 2002; 347:1397-402). Similar to cardiac hypertrophy and hypertension, heart failure is less common in women than in men and the prognosis of manifested heart failure is significantly better in female patients (Luchner A, Brockel U, Muscholl M, Hense HW, Doring A, Riegger GA, Schunkert H. Gender-specific differences of cardiac remodeling in subjects with left ventricular dysfunction: a population-based study. Cardiovasc Res. 2002; 53:720-7).

Estrogens modulate the expression of target genes and hence myocardial as well as vascular function via two distinct nuclear hormone receptors, estrogen receptor α (ERα) and estrogen receptor β (ERβ), acting as ligand dependent transcription factors (Walters P, Green S, Greene G, Krust A, Bornert JM, Jeltsch JM, Staub A, Jensen E, Scrace G, Waterfield M, Chambon P. Cloning of the human estrogen receptor cDNA. Proc Natl Acad Sci USA 1985; 82:7889-7893; Kuiper GG, Enmark E, Pelto-Huikko M, Nilsson S, Gustafsson JA. Cloning of a novel receptor expressed in rat in rat prostate and ovary. Proc Natl Acad Sci USA 1996; 93:5925-5930). ERα as well as ERβ are expressed and functional in vascular cells as well as in cardiac myocytes in several species including mice, rats and humans.
Both of the known estrogen receptor (ER) subtypes, ERα and ERβ, are encoded by distinct genes. They exhibit a high degree of sequence homology. Thus, the amino acid sequence within the hormone binding domain differs by three amino acids only. The two receptor isotypes mediate divergent but also redundant biological effects in several organ systems (Kuiper GG, Carlsson B, Grandien K, Enmark E, Haggblad J, Nilsson S, Gustafsson JA. Comparison of the Ligand Binding Specificity and Transcript Tissue Distribution of Estrogen Receptors α and β Endocrinology 1997; 138:863-870; Jankowski M, Rachelska G, Donghao W, McCann SM, Gutkowska J. Estrogen receptors activate atrial natriuretic peptide in the rat heart. Proc Natl Acad Sci USA 2001; 98:11765-11770).

### Tissue distribution and function of estrogen receptor beta (ERβ)

ERβ has been identified as a second ER subtype in 1995/96 (Kuiper et al. (1996), Proc. Natl. Acad. Sci. 93:5925-5930; Mosselman, Dijkema (1996) Febs Letters 392: 49-53; Tremblay et al. (1997), Molecular Endocrinology 11: 353-365). The expression pattern of ERβ differs from ERα (Kuiper et al. (1996), Endocrinology 138: 863-870). The expression of ERβ is high in rat prostate whereas its expression is low in the uterus. Other organs showing high expression of ERβ are the ovarian granulosa cells (Couse et al. Endocrinology 138, 1997, S. 4612-4613). In addition to that, organs with high expression of ERβ comprize the bone (Onoe Y et al., 1997, Endocrinology 138:4509-4512), the vascular system (Register TC, Adams MR 1998, J Steroid Molec Biol 64: 187-191), the lung, the urogenital tract (Kuiper GJM et al. 1997, Endocrinology 138: 863-870), the gastrointestinal tract (Campbell-Thopson 1997, BBRC 240: 478-483) and the testis (Mosselmann S et al. 1996 Febs Lett 392 49-53). Evidence for the functional importance of ERβ in the vascular system has been provided by the phenotype of ERα, ERβ, and ERα/β-knockout mice. Thus, estradiol exhibits a protecive effect in an artheriosclerosis model (vascular injury response model) in both, ERα and ERβ knockout mice, indicating that the remaining ER isotype mediates the protective effect (Iafrati MD et al. 1997, Nature Medicine 3: 545-548). The protective effect of estradiol is lost in ERα/ERβ double knockout mice (Karas RH et al. 2001, Circ. Res 89: 534-539).

Abnormal vascular function and hypertension has been observed in ERR-deficient mice (Zhu Y et al. 2002, Science 295: 505-508). The data are in support for an essential role for ERβ in the regulation of vascular function and blood pressure.

### ER ligands and their effect on the cardiovascular system

In contrast to the natural ER ligand 17β-estradiol and the conjugated estrogens (the active principle in PREMARIN) used in hormone replacement therapy (HRT), the effects of subtype selective ER agonists in the cardiovascular system have not been studied in detail. Whereas 17β-estradiol as well as conjugated estrogens and synthetic estrogens stimulate the uterine endometrium and therefore are often given in combination with a progestin, isotype selective estrogens are expected to allow estrogen therapy without causing undesired stimulatory effects e.g. on the uterus and the liver. Stimulation of the uterine endometrium with estrogens is supposed to be associated with an increased risk of endometrial carcinoma (Harlap S 1992, Am J Obstet Gynecol 166: 1986-1992).

One class of selective estrogens exhibiting estrogen-like effects on the bone and the vascular system but antiestrogenic effects on the uterus and the breast are ER partial agonists/antagonists. This type of selective estrogens is called ,Selective Estrogen Receptor Modulators' (SERM) (R.F. Kauffman, H.U. Bryant 1995, DNAP 8 (9): 531-539). For SERMs an improvment of endothelial function has been shown, however, beneficial effects of SERMs on blood pressure, cardiac hypertrophy and cardiac output have not been described (Nickenig G, Baumer AT, Grohe C, Kahlert S, Strehlow K, Rosenkranz S, Stablein A, Beckers F, Smits JF, Daemen MJ, Vetter H, Boehm M. Estrogen modulates AT1 receptor gene expression in vitro and in vivo. Circulation 1998; 97:2197-22019).
Recently, ERβ selective estrogens have been identified (e.g in WO 01/77139; PCT/EP03/06172, WO 02/26763; DE 100486349).

DE 10019167 describes 8β-substituted estratrienes as pharmaceutically active ingredients that have *in vitro* a higher affinity to estrogen receptor preparations of rat prostates than to estrogen receptor preparations of rat uteri. DE 10019167 also describes the use of these compounds for treatment of estrogen-deficiency-induced diseases and conditions and particularly for the prevention of cardiovascular diseases, especially vascular diseases such as arteriosclerosis, for inhibition of the proliferation of arterial smooth muscle cells and for the treatment of primary pulmonary high blood pressure.

WO 03/104253 describes 9α-substituted estratrienes as pharmaceutically active ingredients that exhibit *in vitro* a higher affinity to estrogen receptor preparations from rat prostates than to estrogen receptor preparations from rat uteri and *in vivo* preferably a preferential action on the ovary in comparison to the uterus, their production, their therapeutic use and pharmaceutical dispensing forms containing the estratrienes.

### Invention

The present inventors have studied the cardiovascular effects of the selective ERβ agonists 8β-Vinyl-estra- 1 ,3,5(10)-trien-3, 17β-diol and 17β-Fluor-9α-vinyl-estra-1,3,5(10)-trien-3,16α-diol in spontaneously hypertensive rats, an established model system that mimics essential human hypertension and cardiac hypertrophy (Dantas AP, Scivoletto R, Fortes ZB, Nigro D, Carvalho MH. Influence of female sex hormones on endothelium-derived vasoconstrictor prostanoid generation in microvessels of spontaneously hypertensive rats. Hypertension. 1999;34:914-9.). In performing these studies, a significant reduction of systolic as well as diastolic blood pressure in animals treated with the compounds 8β-Vinyl-estra-1,3,5(10)-trien-3,17β-diol and 17β-Fluor-9α-vinyl-estra-1,3,5(10)-trien-3,1 6α-diol was observed.

Surprisingly, the development of cardiac hypertrophy as assessed by morphometry as well as cardiac MRI in vivo was significantly inhibited in these animals upon treatment with 8β-Vinyl-estra-1,3,5(10)-trien-3,17β-diol and 17β-Fluor-9α-vinyl-estra-1,3,5(10)-trien-3,16α-diol. Invasive as well as non-invasive hemodynamic studies indicated a significant improvement of cardiac output in animals treated with the compounds 8β-Vinyl-estra-1,3,5(10)-trien-3,17β-diol and 17β-Fluor-9α-vinyl-estra-1,3,5(10)-trien-3,16α-diol.

Based on these results, it is concluded that the selective ER-β agonists 8β-Vinyl-estra-1,3,5(10)-trien-3,17β-diol and 17β-Fluor-9α-vinyl-estra-1,3,5(10)-trien-3,16α-diol offer a new approach to the prevention and treatment of one or more of the conditions selected from the group of (1) hypertension, (2) cardiac hypertrophy and (3) heart failure in humans.

According to the present invention the ERβ selective agonists 8β-Vinyl-estra-1, 3,5(10)-trien-3 , 17β-diol and 17β-Fluor-9α-vinyl-estra-1,3,5(10)-trien-3, 16α-diol and prodrugs thereof can be used for the production of medicaments for the prevention and treatment of one or more conditions selected from the group of (1) hypertension, (2) cardiac hypertrophy and (3) heart failure.

The compounds are suitable to be used for these indications in females and males.

The ERβ-selective agonists 8β-Vinyl-estra-1,3,5(10)-trien-3,17β-diol and 17β-Fluor-9α-vinyl-estra-1,3,5(10)-trien-3,16α-diol are to be used without the co-administration of additional progestin for the purposes of the invention. This is because the ERβ-selective agonists will not lead to a stimulation of the endometrium which in the case of the use of ERα-agonistic compounds would have to be antagonized by coadministration of a progestin.

The ERβ-selective agonists 8β-Vinyl-estra-1,3,5(10)-trien-3, 17β-diol and 17β-Fluor-9α-vinyl-estra-1,3,5(10)-trien-3,16α-diol are especially suitable to be used for the purposes of the invention for women exhibiting reduced endogenous estrogen levels due to postmenopausal decline of ovarian function, or chemical or surgical castration.

In a further aspect of the invention the ERβ-selective agonists can be administered in combination with (in addition to) antiestrogens and/or aromatase inhibitors which are administered for the treatment of hormone dependent and independent tumors. The purpose of the administration of the ERβ-selective agonist is to prevent detrimental effects of estrogen deprivation on the cardiovascular system, specifically negative effects on blood pressure and cardiac function.

In a still further aspect of the invention the ERβ-selective agonists can be given in combination with antiestrogens, aromatase inhibitors or Selective Estrogen Receptor Modulators (SERM) used for the treatment of prostate hyperplasia in order to prevent detrimental effects of estrogen deprivation on the cardiovascular system, specifically negative effects on blood pressure and cardiac function.

The antiestrogen to be used can be for instance 7α-[9-[(4,4,5,5,5-pentafluoropentyl)sulfinyl]nonyl]estra-1,3,5(10)-triene-3,17β-diol (fulvestrant).

The aromatase inhibitor to be used can be selected from the group of the following compounds: anastrozole, atamestane, fadrozole, formestane, letrozole.

The SERM to be used can be selected from the group of the following compounds: raloxifene, tamoxifene, 5-(4-{5-[(RS)-(4,4,5,5,5-pentafluoropentyl)sulfinyl]pentyloxy} phenyl)-6-phenyl-8,9-dihydro-7H-benzocyclohepten-2-ol (WO 00/03979).

The compounds can be used for the mentioned indications both after oral and parenteral administration.

The amount of a compound with ERβ-agonistic activity that is to be administered varies within a wide range and can cover any effective amount. On the basis of the condition that is to be treated or the effect to be achieved and the type of administration, the amount of the compound that is administered can be 0.001 mg / kg - 100 mg/kg of body weight, preferably 0.003 mg/kg - 30 mg/kg of body weight, per day.
In humans, this corresponds to a dose of 0.080 mg to 8000 mg, preferably 0.240 to 2400 mg, daily.

According to the invention, a dosage unit contains 0.08 mg to 2000 mg of one or both of the compounds with ERβ-agonistic activity

Pharmaceutical compositions or pharmaceutical agents containing compounds with ERβ-agonistic activity to be used according to the invention contain as active ingredient one or both compounds 8β-Vinyl-estra-1,3,5(10)-trien-3,17β-diol and 17β-Fluor-9α-vinyl-estra-1,3,5(10)-trien-3,16α-diol, optionally mixed with other pharmacologically or pharmaceutically active substances. The production of the pharmaceutical agents is carried out in a known way, whereby the known and commonly used pharmaceutical adjuvants as well as other commonly used vehicles and diluents can be used.

As such vehicles and adjuvants, for example, those are suitable that are recommended or indicated in the following bibliographic references as adjuvants for pharmaceutics, cosmetics and related fields: Ullmans EncyklopAdie der technischen Chemie [Ullman's Encyclopedia of Technical Chemistry], Volume 4 (1953), pages 1 to 39; Journal of Pharmaceutical Sciences, Volume 52 (1963), page 918 ff., issued by Czetsch-Lindenwald, Hilfsstoffe for Pharmazie und angrenzende Gebiete [Adjuvants for Pharmaceutics and Related Fields]; Pharm. Ind., Issue 2, 1961, p. 72 and ff.: Dr. H. P. Fiedler, Lexikon der Hilfsstoffe for Pharmazie, Kosmetik und angrenzende Gebiete [Dictionary of Adjuvants for Pharmaceutics, Cosmetics and Related Fields], Cantor KG, Aulendorf in Württemberg 117β-Fluor-9α-vinyl-estra-1,3,5(10)-trien-3,16α-diol 971.

The compounds can be administered orally or parenterally, for example intraperitoneally, intramuscularly, subcutaneously percutaneously or intravenously. The compounds can also be implanted subcutaneuosly in the form of slow release systems into the tissue.

For oral administration, capsules, pills, tablets, coated tablets, etc., are suitable. In addition to the active ingredient, the dosage units can contain a pharmaceutically compatible vehicle, such as, for example, starch, sugar, sorbitol, gelatin, lubricant, silicic acid, talc, etc..

For parenteral administration, the active ingredients can be dissolved or suspended in a physiologically compatible diluent. As diluents, very often oils with or without the addition of a solubilizer, a surfactant, a suspending agent or an emulsifying agent are used. Examples of oils that are used are olive oil, peanut oil, cottonseed oil, soybean oil, castor oil and sesame oil.

The compounds can also be used in the form of a depot injection or an implant preparation, which can be formulated so that a delayed release of active ingredient is made possible.
As inert materials, implants can contain, for example, biodegradable polymers, or synthetic silicones such as, for example, silicone rubber. In addition, for percutaneous administration, the active ingredients can be added to, for example, a patch.
For the production of intravaginal systems (e.g., vaginal rings) or intrauterine systems (e.g., pessaries, coils, IUDs, Mirena^{®}) that are loaded with a compound with ERβ-agonistic activity for local administration, various polymers are suitable, such as, for example, silicone polymers, ethylene vinyl acetate, polyethylene or polypropylene.
To achieve better bio-availability of the active ingredient, the compounds can also be formulated as cyclodextrin clathrates. For this purpose, the compounds are reacted with α, β-, or γ-cyclodextrin or derivatives of the latter (PCT/EP95/02656).

According to the invention, the compounds with ERβ-agonistic activity can also be encapsulated with liposomes.

### Brief description of the drawings

In the following figures ERβ-agonist stands for 8β-Vinyl-estra-1,3,5(10)-trien-3,17β-diol and ERα-agonist stands for 3,17β-Dihydroxy-19-nor-17α-pregna-1,3,5(10)-triene-21,16α-lactone.
Figure 1 is a graph showing the effect of treatment with different ER ligands on uterine weight in ovarectomized (ovx) SHR.
Figure 2 is a graph showing the effect of treatment with different ER ligands on body weight of ovx SHR.
Firgure 3 is a graph showing the Effect of treatment with ER ligands on blood pressure (SAP) of ovx SHR (mean of two studies).
Figure 4 is a graph showing the effect of treatment with different ER ligands on cardiac ouput in ovx SHR (mean of two studies).
Figure 5 is a graph showing the effect of treatment with different ER ligands on stroke volume in ovx SHR (mean of two studies).
Figure 6 is a graph showing the effect of treatment with different ER ligands on heart weight in ovx SHR (mean of two studies).

### Methods

### Estrogen receptor binding studies

The binding affinity of 8β-Vinyl-estra-1,3,5(10)-trien-3,17β-diol and 17β-Fluor-9α-vinyl-estra-1,3,5(10)-trien-3,16α-diol was determined by competition experiments using [3H]-estradiol as a ligand. Receptor containing cytosol was prepared from rat prostate (ERβ containing cytosol) and rat uterus (ERα containing cytosol).

The preparation of rat uterus cytosol and the binding study were performed as described (Stack, Gorski 1985, Endocrinology 117, 2024-2032) with some modifications as described by Fuhrmann et al. (Fuhrmann U. et al. 1995, Contraception 51: 45-52).
The preparation of rat prostate cytosol and the binding study was performed as described (Testas J. et al., 1981, Endocrinology 109: 1287-1289).

8β-Vinyl-estra-1,3,5(10)-trien-3,17β-diol and 17β-Fluor-9α-vinyl-estra-1,3,5(10)-trien-3,16α-diol exhibit higher binding affinity to the rat prostate compared to rat uterus estrogen receptor, ERβ being the predominant ER in rat prostate, ERα in rat uterus. In accordance with this assumption, it was found that 8β-Vinyl-estra-1,3,5(10)-trien-3,17β-diol and 17β-Fluor-9α-vinyl-estra-1,3,5(10)-trien-3,16α-diol exhibit higher affinity to human ERβ compared to ERα, the human receptors being expressed by the Baculovirus/SF-9 - expression system.

### Study of the effect of the ERβ agonists 8β-Vinyl-estra-1,3,5(10)-trien-3 17β-diol and 17β-Fluor-9α-vinyl-estra-1,3,5(10)-trien-3,16α-diol on blood pressure and cardiac function in spontaneous hypertensive rats (SHR)

The effects of the ERβ-agonists on blood pressure (BP) in ovariectomized SHR, a widely accepted animal model of hypertensive heart disease, were investigated in three independent studies. Estradiol and the steroidal ERα agonist 3, 17β-Dihydroxy-19-nor-17α-pregna-1,3,5(10)-triene-21,16α-lactone were used as references.

### Animal housing and care:

Female spontaneously hypertensive rats (SHR-Nico; 12 weeks old; Charles River Laboratories) were used for the experiment. All animals were kept under standard conditions (eg 12 hr ON / OFF light cycle, commercial diet and water ad libitum). Animals were randomly assigned to different treatment groups as outlined below. All animals were marked individually by ear tag.

### Ovarectomy and treatment:

Female rats (SHR; 12 weeks old) were ovarectomized (ovx) and treated for 4 weeks starting one day after ovx according to the protocol outlined below.

### Drugs:

All compounds were injected daily using ethanol (EtOH) / peanut oil 4+1 as the vehicle.

### Study protocol and hemodynamic measurements

| **Group** | **Treatment phase** | **animals** / **group** |
|---|---|---|
| 1. sham OP | 12 to 16 weeks of age | 10 |
| 2. ovx, EtOH / peanut oil | 12 to 16 weeks of age | 10 |
| 3. ovx, 17β-estradiol (2µg/kg bw/d) | 12 to 16 weeks of age | 10 |
| 4. ovx, 3,17β-Dihydroxy-19-nor-17α-pregna-1,3,5(10)-triene-21,16α-lactone (30 µg / kg BW / d / sc) | 12 to 16 weeks of age | 10 |
| 5. ovx, ERβ agonist 8β-Vinyl-estra-1,3,5(10)-trien-3,17β-diol (30 µg / kg BW / d / sc) | 12 to 16 weeks of age | 10 |
| [or 17β-Fluor-9α-vinyl-estra-1,3,5(10)-trien-3,16α-diol (30 µg / kg BW / d / sc), respectively] | | |

12 weeks old female SHR (Charles River^{™}) were ovariectomized or sham-operated. Starting one day after ovariectomy animals were randomly selected for a daily subcutaneous injection of 17β-estradiol (E2, 2µg/kg body weight/d), the selective ERα agonist 3,17β-Dihydroxy-19-nor-17α-pregna-1,3,5(10)-triene-21,16α-lactone (WO 02/26763) (30µg/kg body weight/d), the selective ERβ agonist 8β-Vinyl-estra-1,3,5(10)-trien-3,17β-diol (or 17β-Fluor-9α-vinyl-estra-1,3,5(10)-trien-3,16α-diol, respectively 30µg/kg body weight) or placebo. All animals had free access to standard rat chow and water. E2, 3,17β-Dihydroxy-19-nor-17α-pregna-1,3,5(10)-triene-21,16α-lactone and 8β-Vinyl-estra-1,3,5(10)-trien-3,17β-diol (or 17β-Fluor-9α-vinyl-estra-1,3,5(10)-trien-3,16α-diol) were dissolved in ethanol and injected daily using peanut oil as a carrier substance, placebo animals received ethanol/peanut oil alone. After 4 weeks treatment hemodynamic parameters were measured as described previously (Fraccarollo D, Hu K, Galuppo P, Gaudron P, Ertl G. (1997), Circulation. 1997;96:3963-3973) and animals were euthanized.

### In vivo hemodynamics:

The following parameters were determined: Systolic, diastolic, mean blood pressure; left ventricular ejection fraction; systolic and diastolic left ventricular pressure as well as the velocity of pressure increase and decrease in the left ventricle; cardiac output, cardiac index, heart rate; systemic vascular resistance. All measurements were performed at rest under isofluran anesthesia.

### Cardiac hypertrophy:

Cardiac hypertrophy in all animals was determined by measuring total heart weight and subsequent normalization to tibia length.

### Uterus weight:

Uterus fresh weight was measured as an indicator of classical ERα-mediated estrogen action.

### Statistics

Values are expressed as mean ± SEM of parameters from different animals. Statistical analysis was performed by one-way analysis of variance (ANOVA) followed by a post hoc Bonferroni test. P values < 0.05 were considered statistically significant.

### Results

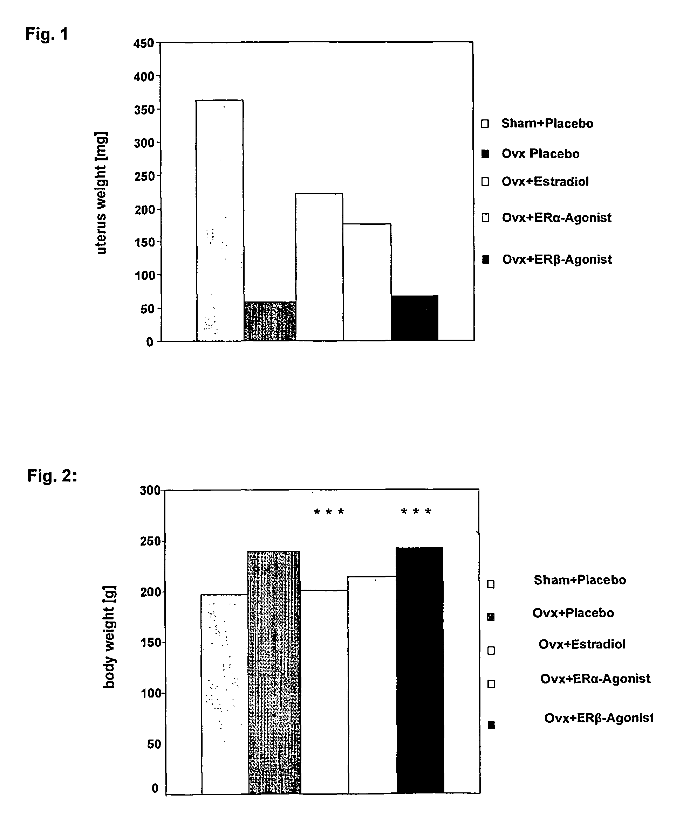

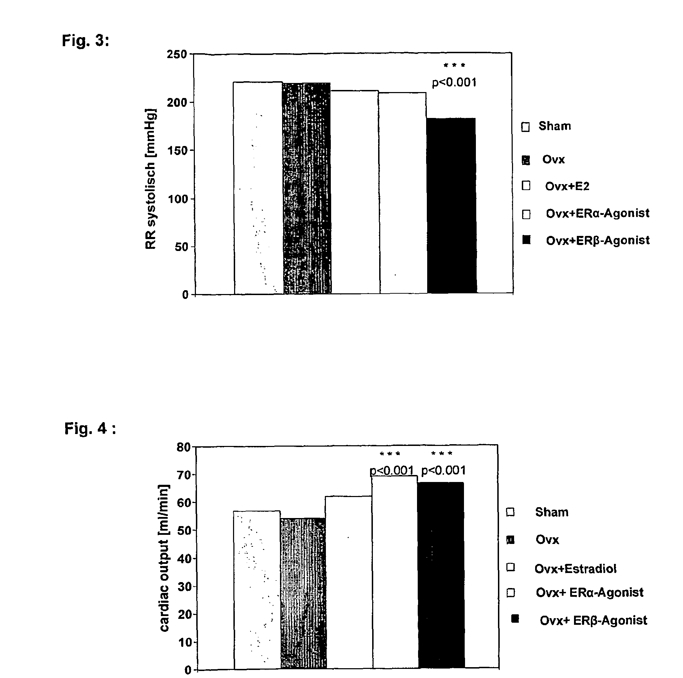

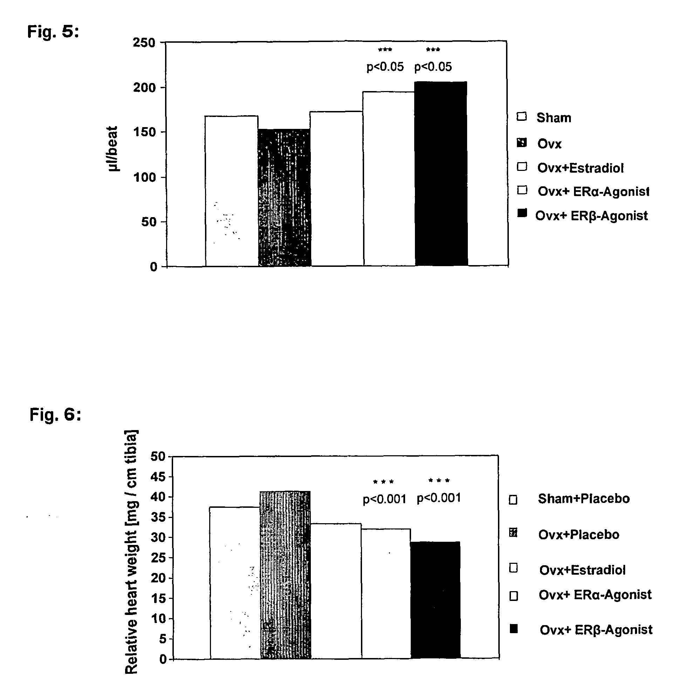

The results provide clear evidence for favorable effects of 8β-Vinyl-estra-1,3,5(10)-trien-3,17β-diol and 17β-Fluor-9α-vinyl-estra-1,3,5(10)-trien-3,16α-diol in an animal model of hypertension, cardiac hypertrophy and heart failure (SHR rats).
• 8β-Vinyl-estra-1,3,5(10)-trien-3,17β-diol (dose: 30 µg/kg/d), in contrast to the natural estrogen estradiol (2 µg/kg/d) and the ERα agonist 3,17β-Dihydroxy-19-nor-17α-pregna-1,3,5(10)-triene-21,16α-lactone (dose: 30 µg/kg/d), did neither cause stimulation of uterine growth nor affect the ovx-induced adipose phenotype of ovx SHR (figures 1, 2).
• 8β-Vinyl-estra-1 ,3,5(10)-trien-3,17β-diol lowered blood pressure levels in SHR compared to vehicle treated (placebo) animals. The compound was more effective than estradiol and the ERα agonist 3,17β-Dihydroxy-19-nor-17α-pregna-1,3,5(10)-triene-21,16α-lactone (figure 3).
• 8β-Vinyl-estra-1,3,5(10)-trien-3,17β-diol augmented global left ventricular performance indices in estradiol-deficient SHR rats such as cardiac output, left-ventricular stroke volume (figures 4,5) and cardiac index.
• 8β-Vinyl-estra-1,3,5(10)-trien-3,17β-diol, unlike estradiol and the ERα agonist 3,17β-Dihydroxy-19-nor-17α-pregna-1,3,5(10)-triene-21,16α-lactone, caused an inhibitory effect on cardiac hyperthrophy as indicated by a reduction in heart weight compared to placebo treated rats (figure 6).
• 17β-Fluor-9α-vinyl-estra-1,3,5(10)-trien-3,16α-diol (30 µg/kg/d) was tested in an independent experiment using the same study design. The ERβ agonist exhibited a similar pharmacological profile as 8β-Vinyl-estra-1,3,5(10)-trien-3,17β-diol in SHR (data not shown).

## Claims

1. Use of the ERβ agonists 8β-Vinyl-estra-1,3,5(10)-trien-3,17β-diol and/or 17β-Fluor- 9α-vinyl-estra-1,3,5(10)-trien-3,16α-diol for production of medicaments for the treatment of hypertensive heart disease.

2. Use of the ERβ agonists 8β-Vinyl-estra-1,3,5(10)-trien-3,17β-diol and/or 17β-Fluor-9α-vinyl-estra-1,3,5(10)-trien-3,16α-diol according to claim 1 for production of medicaments for the treatment of more than one of the conditions selected from the group of (1) hypertension, (2) cardiac hypertrophy, and (3) heart failure.

3. Use of the ERβ agonists 8β-Vinyl-estra-1,3,5(10)-trien-3,17β-diol for production of medicaments for the treatment of one or more of the conditions, selected from the group of (1) hypertension, (2) cardiac hypertrophy and (3) heart failure.

4. Use of the ERβ agonists 17β-Fluor- 9α-vinyl-estra-1,3,5(10)-trien-3,16α-diol for production of medicaments for the treatment of one or more of the conditions, selected from the group of (2) cardiac hypertrophy and (3) heart failure.

5. Use of the ERβ agonists 8β-Vinyl-estra-1 ,3,5(10)-trien-3, 17β-diol and/or 17β-Fluor-9α-vinyl-estra-1,3,5(10)-trien,10α-diol according to claim 1 for the treatment of estrogen deficiency-related hypertensive heart disease.

6. Use according to claim 5 for treatment of vascular dysfunction related to hypertension.

7. Use according to claim 6 for treatment of vascular dysfunction related to impaired vascular NO production.

8. Use according to claim 6 for treatment of heart failure.

9. Use according to claim 6 for treatment of cardiac hypertrophy.

10. Use of the ERβ agonists 8β-Vinyl-estra-1,3,5(10)-trien-3,17β-diol and/or 17β-Fluor-9α-vinyl-estra-1,3,5(10)-trien-3,16α-diol according to anyone of claims 5 to 9 in postmenopausal women.

11. Use of the ERβ agonists 8β- Vinyl-estra-1,3,5(10)-trien-3,17α-diol/or and 17β-Fluor-9α-vinyl-estra-1,3,5(10)-trien-3,16α-diol according to anyone of claims 5 to 10 in ovariectomized women.

12. Use of the ERβ agonists 8β-Vinyl-estra-1,3,5(10)-trien-3,17β-diol and/or 17p-Fluor-9α-vinyl-estra-1,3,5(10)-trien-3,16α-diol according to anyone of claims 5 to 10 in women treated with GnRH agonists or GnRH antagonists.

13. Use of the ERβ agonists 8β-Vinyl-estra-1,3,5(10)-trien-3,17β-diol and/or 17p-Fluor-9α-vinyl-estra-1,3,5(10)-trien-3,16α-diol according to anyone of claims 5 to 10 in women treated with at least one compound selected of the group of antiestrogens, SERMs or aromatase inhibitors.

14. Use of the ERβ agonists 8β-Vinyl-estra-1,3,5(10)-trien-3,17β-diol and/or 17β-Fluor-9α-vinyl-estra-1,3,5(10)-trien-3,16α-diol according to anyone of claims 5 to 9 in males treated with at least one compound selected of the group of antiestrogens, SERMs, GnRH agonists, GnRH antagonists, aromatase inhibitors or progestins.

15. Use according to claim 8 to improve decreased cardiac output and/or stroke volume in heart failure.

16. Use of the ERβ agonists 8β-Vinyl-estra-1,3,5(10)-trien-3,17β-diol and/or 17β-Fluor-9α-vinyl-estra-1,3,5(10)-trien-3,16α-diol according to claim 15 in postmenopausal women.

17. Use of the ERβ agonists 8β-Vinyl-estra-1.3,5(10)-trien-3.17β-diol and/or 17β-Fluor-9α-vinyl-estra-1,3,5(10)-trien-3,16α-diol according to claim 16 in ovariectomized women.

18. Use according to anyone of claims 15 to 17 in women treated with GnRH agonists or GnRH antagonists.

19. Use according to anyone of claims 15 to 17 in women treated with at least one compound selected of the group of antiestrogens, SERMs or aromatase inhibitors.

20. Use of the ERβ agonists 8β-Vinyl-estra-1,3,5(10)-trien-3,17β-diol and/or 17β-Fluor-9α-vinyl-estra-1,3,5(10)-trien-3,16α-diol according to claim 15 or 16 in males treated with at least one compound selected of the group of the antiestrogens, SERMs, GnRH agonists, GnRH antagonists, aromatase inhibitors or progestins.

21. Use of the ERβ agonists 8β-Vinyl-estra-1,3,5(10)-trien-3,17β-diol and/or 17β-Fluor-9α-vinyl-estra-1,3,5(14)-trien-3,16α-diol according to claim 1 for the treatment of hypertension related to pregnancy.

## Patentansprüche

1. Verwendung der ERβ-Agonisten 8β-Vinylestra-1,3,5(10)-trien-3,17β-diol und/oder 17β-Fluor-9α-vinylestra-1,3,5(10)-trien-3,16α-diol zur Herstellung von Arzneimitteln zur Behandlung von hypertonischer Herzerkrankung.

2. Verwendung der ERβ-Agonisten 8β-Vinylestra-1,3,5(10)-trien-3,17β-diol und/oder 17β-Fluor-9α-vinylestra-1,3,5(10)-trien-3,16α-diol nach Anspruch 1 zur Herstellung von Arzneimitteln zur Behandlung von mehr als einem der Zustände, ausgewählt aus der Gruppe mit (1) Hypertonie, (2) Herzhypertrophie und (3) Herzversagen.

3. Verwendung des ERβ-Agonisten 8β-Vinylestra-1,3,5(10)-trien-3,17β-diol zur Herstellung von Arzneimitteln zur Behandlung von einem oder mehreren der Zustände, ausgewählt aus der Gruppe mit (1) Hypertonie, (2) Herzhypertrophie und (3) Herzversagen.

4. Verwendung des ERβ-Agonisten 17β-Fluor-9α-vinylestra-1,3,5(10)-trien-3,16α-diol zur Herstellung von Arzneimitteln zur Behandlung von einem oder mehreren der Zustände, ausgewählt aus der Gruppe mit (2) Herzhypertrophie und (3) Herzversagen.

5. Verwendung der ERβ-Agonisten 8β-Vinylestra-1,3,5(10)-trien-3,17β-diol und/oder 17β-Fluor-9α-vinylestra-1,3,5(10)-trien-3,16α-diol nach Anspruch 1 zur Behandlung von hypertonischer Herzerkrankung in Verbindung mit Estrogenmangel.

6. Verwendung nach Anspruch 5 zur Behandlung von vaskulärer Dysfunktion in Verbindung mit Hypertonie.

7. Verwendung nach Anspruch 6 zur Behandlung von vaskulärer Dysfunktion in Verbindung mit gestörter vaskulärer NO-Produktion.

8. Verwendung nach Anspruch 6 zur Behandlung von Herzversagen.

9. Verwendung nach Anspruch 6 zur Behandlung von Herzhypertrophie.

10. Verwendung der ERβ-Agonisten 8β-Vinylestra-1,3,5(10)-trien-3,17β-diol und/oder 17β-Fluor-9α-vinylestra-1,3,5(10)-trien-3,16α-diol nach einem der Ansprüche 5 bis 9 bei postmenopausalen Frauen.

11. Verwendung der ERβ-Agonisten 8β-Vinylestra-1,3,5(10)-trien-3,17β-diol und/oder 17β-Fluor-9α-vinylestra-1,3,5(10)-trien-3,16α-diol nach einem der Ansprüche 5 bis 10 bei ovarektomierten Frauen.

12. Verwendung der ERβ-Agonisten 8β-Vinylestra-1,3,5(10)-trien-3,17β-diol und/oder 17β-Fluor-9α-vinylestra-1,3,5(10)-trien-3,16a-diol nach einem der Ansprüche 5 bis 10 bei mit GnRH-Agonisten oder GnRH-Antagonisten behandelten Frauen.

13. Verwendung der ERβ-Agonisten 8β-Vinylestra-1,3,5(10)-trien-3,17β-diol und/oder 17β-Fluor-9α-vinylestra-1,3,5(10)-trien-3,16α-diol nach einem der Ansprüche 5 bis 10 bei Frauen, die mit mindestens einer Verbindung, ausgewählt aus der Gruppe mit Antiestrogenen, SERM oder Aromataseinhibitoren, behandelt werden.

14. Verwendung der ERβ-Agonisten 8β-Vinylestra-1,3,5(10)-trien-3,17β-diol und/oder 17β-Fluor-9α-vinylestra-1,3,5(10)-trien-3,16α-diol nach einem der Ansprüche 5 bis 9 bei Männern, die mit mindestens einer Verbindung, ausgewählt aus der Gruppe mit Antiestrogenen, SERM, GnRH-Agonisten, GnRH-Antagonisten, Aromataseinhibitoren oder Progestinen, behandelt werden.

15. Verwendung nach Anspruch 8 zur Verbesserung eines verringerten Herzausstoßes und/oder Schlagvolumens bei Herzversagen.

16. Verwendung der ERβ-Agonisten 8β-Vinylestra-1,3,5(10)-trien-3,17β-diol und/oder 17β-Fluor-9α-vinylestra-1,3,5(10)-trien-3,16α-diol nach Anspruch 15 bei postmenopausalen Frauen.

17. Verwendung der ERβ-Agonisten 8β-Vinylestra-1,3,5(10)-trien-3,17β-diol und/oder 17β-Fluor-9α-vinylestra-1,3,5(10)-trien-3,16α-diol nach Anspruch 16 bei ovarektomierten Frauen.

18. Verwendung nach einem der Ansprüche 15 bis 17 bei mit GnRH-Agonisten oder GnRH-Antagonisten behandelten Frauen.

19. Verwendung nach einem der Ansprüche 15 bis 17 bei Frauen, die mit mindestens einer Verbindung, ausgewählt aus der Gruppe mit Antiestrogenen, SERM oder Aromataseinhibitoren, behandelt werden.

20. Verwendung der ERβ-Agonisten 8β-Vinylestra-1,3,5(10)-trien-3,17β-diol und/oder 17β-Fluor-9α-vinylestra-1,3,5(10)-trien-3,16α-diol nach Anspruch 15 oder 16 bei Männern, die mit mindestens einer Verbindung, ausgewählt aus der Gruppe mit Antiestrogenen, SERM, GnRH-Agonisten, GnRH-Antagonisten, Aromataseinhibitoren oder Progestinen, behandelt werden.

21. Verwendung der ERβ-Agonisten 8β-Vinylestra-1,3,5(10)-trien-3,17β-diol und/oder 17β-Fluor-9α-vinylestra-1,3,5(10)-trien-3,16α-diol nach Anspruch 1 zur Behandlung von Hypertonie in Verbindung mit Schwangerschaft.

## Revendications

1. Utilisation des agonistes d'ERβ, le 8β-vinyl-estra-1,3,5(10)-triène-3,17β-diol et/ou le 17β-fluor-9α-vinyl-estra-1,3,5(10)-triène-3,16α-diol, pour la production de médicaments destinés au traitement de maladies cardiaques hypertensives.

2. Utilisation des agonistes d' ERβ, le 8β-vinyl-estra-1,3,5(10)-triène-3,17β-diol et/ou le 17β-fluor-9α-vinyl-estra-1,3,5(10)-triène-3,16α-diol selon la revendication 1, pour la production de médicaments destinés au traitement de plus de l'une des affections : choisies dans le groupe constitué de (1) l'hypertension, (2) l'hypertrophie cardiaque et (3) l'insuffisance cardiaque.

3. Utilisation des agonistes d' ERβ, le 8β-vinyl-estra-1,3,5(10)-triène-3,17β-diol, pour la production de médicaments destinés au traitement d'une ou plusieurs des affections choisies dans le groupe constitué de (1) l'hypertension, (2) l'hypertrophie cardiaque et (3) l'insuffisance cardiaque.

4. Utilisation des agonistes d' ERβ, le 17β-fluor-9α-vinyl-estra-1,3,5(10)-triène-3,16α-diol, pour la production de médicaments destinés au traitement d'une ou plusieurs des affections choisies dans le groupe constitué de (2) l'hypertrophie cardiaque et (3) l'insuffisance cardiaque.

5. Utilisation des agonistes d'ERβ, le 8β-vinyl-estra-1,3,5(10) -triène-3,17β-diol et/ou le 17β-fluor-9α-vinyl-estra-1,3,5(10)-triène-3,16α-diol, selon la revendication 1, pour le traitement de maladies cardiaques hypertensives liées à la déficience en oestrogènes.

6. Utilisation selon la revendication 5, pour le traitement du dysfonctionnement vasculaire lié à l'hypertension.

7. Utilisation selon la revendication 6, pour le traitement du dysfonctionnement vasculaire lié à la production vasculaire altérée de NO.

8. Utilisation selon la revendication 6, pour le traitement de l'insuffisance cardiaque.

9. Utilisation selon la revendication 6, pour le traitement de l'hypertrophie cardiaque.

10. Utilisation des agonistes d'ERβ, le 8β-vinyl-estra-1,3,5(10)-triène-3,17β-diol et/ou le 17β-fluor-9α-vinyl-estra-1,3,5(10)-triène-3,16α-diol, selon l'une quelconque des revendications 5 à 9, chez la femme post-ménopausée.

11. Utilisation des agonistes d' ERβ, le 8β-vinyl-estra-1,3,5(10)-triène-3,17β-diol et/ou le 17β-fluor-9α-vinyl-estra-1,3,5(10)-triène-3,16α-diol, selon l'une quelconque des revendications 5 à 10, chez la femme ovarectimisée.

12. Utilisation des agonistes d' ERβ, le 8β-vinyl-estra-1,3,5(10)-triène-3,17β-diol et/ou le 17β-fluor-9α-vinyl-estra-1,3,5(10)-triène-3,16α-diol, selon l'une quelconque des revendications 5 à 10, chez la femme traitée avec des agonistes de la GnRH ou des antagonistes de la GnRH.

13. Utilisation des agonistes d'ERβ, le 8β-vinyl-estra-1,3,5(10)-triène-3,17β-diol et/ou le 17β-fluor-9α-vinyl-estra-1,3,5(10)-triène-3,16α-diol, selon l'une quelconque des revendications 5 à 10, chez la femme traitée avec au moins un composé choisi dans le groupe constitué des anti-oestrogènes, des SERM, ou des inhibiteurs de l'aromatase.

14. Utilisation des agonistes d'ERβ, le 8β-vinyl-estra-1,3,5(10)-triène-3,17β-diol et/ou le 17β-fluor-9α-vinyl-estra-1,3,5(10)-triène-3,16α-diol, selon l'une quelconque des revendications 5 à 9, chez le mâle traité avec au moins un composé choisi dans le groupe constitué des anti-oestrogènes, des SERM, des agonistes de la GnRH, des antagonistes de la GnRH, des inhibiteurs de l'aromatase ou des progestines.

15. Utilisation selon la revendication 8, pour améliorer un débit cardiaque et/ou un volume systolique réduits lors d'une insuffisance cardiaque.

16. Utilisation des agonistes d' ERβ, le 8β-vinyl-estra-1,3,5(10)-triène-3,17β-diol et/ou le 17β-fluor-9α-vinyl-estra-1,3,5(10)-triène-3,16α,-diol, selon la revendication 15, chez la femme post-ménopausée.

17. Utilisation des agonistes d' ERβ, le 8β-vinyl-estra-1,3,5(10)-triène-3,17β-diol et/ou le 17β-fluor-9α-vinyl-estra-1,3,5(10)-triène-3,16α-diol, selon la revendications 16, chez la femme ovarectimisée.

18. Utilisation selon l'une quelconque des revendications 15 à 17, chez la femme traitée avec des agonistes de la GnRH ou des antagonistes de la GnRH.

19. Utilisation selon l'une quelconque des revendications 15 à 17, chez la femme traitée avec au moins un composé choisi dans le groupe constitué des anti-oestrogènes, des SERM ou des inhibiteurs de l'aromatase.

20. Utilisation des agonistes d'ERβ, le 8β-vinyl-estra-1,3,5(10)-triène-3,17β-diol et/ou le 17β-fluor-9α-vinyl-estra-1,3,5(10)-triène-3,16α-diol, selon la revendication 15 ou 16, chez le mâle traité avec au moins un composé choisi dans le groupe constitué des anti-oestrogènes, des SERM, des agonistes de la GnRH, des antagonistes de la GnRH, des inhibiteurs de l'aromatase ou des progestines.

21. Utilisation des agonistes d'ERβ, le 8β-vinyl-estra-1,3,5(10)-triène-3,17β-diol et/ou le 17β-fluor-9α-vinyl-estra-1,3,5(10)-triène-3,16α-diol, selon la revendication 1, pour le traitement de l'hypertension liée à la grossesse.
